## Europäisches Patentamt

## European Patent Office

## Office européen des brevets

(19)

(11) **EP 0 919 805 A1**

(12) **EUROPÄISCHE PATENTANMELDUNG**

(43) Veröffentlichungstag:
02.06.1999 Patentblatt 1999/22

(51) Int. Cl.⁶: **G01N 22/00**

(21) Anmeldenummer: 98121810.0

(22) Anmeldetag: 17.11.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 28.11.1997 DE 29721039 U

(71) Anmelder: Berthold GmbH & Co. KG
75323 Bad Wildbad (DE)

(72) Erfinder:
• Bürk, Oliver
75378 Bad Liebenzell (DE)

• Reuter, Wilfried, Dr.
75334 Straubenhardt (DE)
• Klute, Ulrich
75305 Neuenbürg (DE)
• Weber, Jean-Marie
68130 Altkirch (FR)

(74) Vertreter:
Frank, Gerhard, Dipl.-Phys. et al
Patentanwälte
Mayer, Frank, Reinhardt,
Schwarzwaldstrasse 1A
75173 Pforzheim (DE)

(54) **Vorrichtung zur Transmissionsmessung mit Hilfe von Mikrowellen**

(57)  Zur kontinuierlichen Bestimmung der Konzentration von Zuckersaft während des Kochprozesses in einem Kochapparat beim Kristallisationsvorgang wird eine Vorrichtung zur Transmissionsmessung mit Hilfe von Mikrowellen vorgeschlagen. Die Vorrichtung weist zwei in das Meßgut einzubringende, als Sende- und Empfangsantenne arbeitende Einrichtungen auf. Diese Einrichtungen sind Hohlleiter (10,20), die zur Abstrahlung der Mikrowellen geeignete Fenster (11,21) aufweisen.

FIG.1

Printed by Xerox (UK) Business Services
2.16.7/3.6

**Beschreibung**

<u>Gebiet der Erfindung</u>

Die Erfindung betrifft eine Vorrichtung zur Transmissionsmessung mit Hilfe von Mikrowellen.

Mikrowellenmeßverfahren werden seit vielen Jahren regelmäßig in der industriellen Prozeßkontrolle eingesetzt. Zu den typischen Applikationen gehören z.B. die Feuchtebestimmung in Schüttgütern oder die Füllstandskontrolle in großen Tanks und Behältern. Auch für die Bestimmung von Dichte und Konzentration von festen und flüssigen Meßgütern wird dieses Verfahren herangezogen. Abstrahlung und Empfang der Mikrowellen geschieht mittels sogenannter Applikatoren, welche im wesentlichen in vier Anordnungen eingesetzt werden: Transmissionsverfahren, Reflexionsverfahren, Probenmessung in einem Resonator und Messgut als Leitungsabschluß. Während die beiden ersten Anordnungen hauptsächlich für online Messungen eingesetzt werden, finden die letzten Anwendung für Einzelprobenuntersuchungen.

Die Bestimmung von physikalischen Parametern wie z.B. Dichte oder Konzentration von Meßgütern in geschlossenen metallischen Behältern erfordert grundsätzlich die Einbringung des Applikators in den Behälter, z.B. über eine Flanschankopplung, so daß eine direkte Wechselwirkung der elektromagnetischen Welle mit dem Meßgut stattfinden kann. Eine Bestimmung der Meßgröße erfordert weiterhin im online Betrieb die ständige Erfassung einer repräsentativen Probe des zu messenden Gutes durch die Mikrowellen.

Der Einsatz von Mikrowellenmeßverfahren zur Ermittlung von physikalischen Parametern wie Dichte, Konzentration oder Feuchte eines Meßgutes hat gegenüber konventionellen Meßmethoden wie Leitfähigkeits- oder Kapazitätsmessungen mehrere Vorteile. Mit zunehmender Frequenz oberhalb von 1 GHz werden der störende Einfluß von z.B. Ionenleitfähigkeit in der Meßprobe oder Kontaktierungsprobleme zunehmend vernachlässigbar. Mit modernen Streifenleiterschaltungen können sehr kompakte Mikrowellenmodule, wie z.B. Synthysizer, Modulatoren oder Demodulatoren mit einem großen Dynamikbereich bezüglich Dämpfung von bis zu 80 dB und Phasenverschiebungsauflösungen bis zehntel Grad erreicht werden.

Das Mikrowellenmeßverfahren wird zurückgeführt auf die Bestimmung der dielektrischen Eigenschaften eines Meßgutes, das durch den Applikator auf geeignete Art und Weise mit einer elektromagnetischen Welle beaufschlagt wird. An die Applikatorform gebunden sind entsprechende auswertbare Mikrowellenmeßgrößen, in diesem Falle Dämpfung und Phasenverschiebung der ins Meßgut eindringenden elektromagnetischen Welle.

Befindet sich ein Dielektrikum in einem Wechselfeld, so können bei hinreichend niedrigen Frequenzen die Dipole dem Feld folgen. Elektrisches Feld und Polarisation des Mediums befinden sich in Phase. Können bei weiterer Erhöhung der Frequenz die Dipole dem äußeren Feld nicht mehr folgen, so tritt eine Phasenverschiebung zwischen der Polarisation und dem elektrischen Feld ein. Dadurch treten Verluste auf, die zur Erwärmung der Probe führen, und der Wert der Dielektrizitätskonstanten (DK) wird kleiner (Dispersion). Eine Beschreibung des Zusammenhangs zwischen elektrischem Wechselfeld E und der Polarisation P wird durch die Einführung der komplexen DK

$$\varepsilon = \varepsilon' - j\varepsilon''$$

ermöglicht. Dabei beschreibt $\varepsilon'$ den Anteil von P, der mit E in Phase ist und geht für die Frequenz f = 0 in die statische Dielektrizitätskonstante über. $\varepsilon''$ beschreibt den Anteil von P, der dem Feld um 90° nachhinkt und ist damit ein Maß für die dielektrischen Verluste.

Diese Ausführung legt nahe, daß im Mikrowellenbereich eine Konzentrationsbestimmung sowohl auf der Messung des Realteils ' als auch des Imaginärteiles $\varepsilon''$ der komplexen DK bzw. von Größen, die mit diesen verknüpft sind, beruhen kann. Da die komplexe DK stark von den physikalischen Eigenschaften des Meßguts, wie z.B. Konzentration, und von der gewählten Hochfrequenz abhängt, sollte eine Frequenz ausgesucht werden, bei welcher der Einfluß der zu messenden Konzentration auf die resultierende komplexe DK der Probe möglichst groß ist. Zur Erhöhung der Meßgenauigkeit können die betrachteten Meßgrößen auch über einen größeren Frequenzbereich gemittelt werden.

Da zur Bestimmung von $\varepsilon'$ und $\varepsilon''$ zwei voneinander unabhängige Messungen notwendig sind, wird ein komplexer Transmissionsfaktor t definiert, der in den Betrag T und die Phase B zerlegt werden kann. Dabei ist t definiert als das Verhältnis der elektrischen Feldstärke der durch die Probe transmittierten Welle $E_t$ zur einfallenden Welle $E_j$:

$$t = T \cdot e^{jB} = E_t / E_j$$

Bei der Bestimmung der komplexen DK $\varepsilon$ aus T und B muß sichergestellt werden, daß die elektromagnetische Welle ausreichend durch das Meßgut gedämpft wird, so daß die Dämpfung beeinflussenden Reflektionen an den Grenzübergängen vernachlässigbar werden können.

<u>Stand der Technik</u>

Eine gattungsgemäße Vorrichtung zur Transmissionsmessung mit Hilfe von Mikrowellen ist aus dem Gebrauchsmuster DE 296 17 488 U1 bekannt; bei diesem "Tauchsensor" werden zwei direkt in das Meßmedium eintauchende Antennen benutzt, die auf einer gemeinsamen Massefläche montiert sind. Hierbei wirkt eine Antenne als Sendereinrichtung, die andere Antenne als Empfangseinrichtung; beide Antennen sind als Monopolstrahler ausgeführt und werden mit Hilfe eines Tauchrohrs an der geeigneten Meßstelle innerhalb eines Kochapparates positioniert, wobei als typische Anwendung dieser Lösung die Führung des Sudprozesses von Verdampfungskristallisatoren in der Zuckerindustrie angegeben wird. Dazu werden die genannten Antennen mit dem Tauchrohr in einem Kochapparat eingebaut und mit Hilfe von Transmissionsmessungen kann die Konzentration des Zuckersaftes während des ablaufenden Kochprozesses kontinuierlich bestimmt werden, wie dies im Grundsatz einleitend beschrieben ist.

Diese vorbekannte Lösung weist jedoch aufgrund ihrer spezifischen Konstruktionsmerkmale einige gravierende Nachteile auf, die eine ordnungsgemäße und präzise Messung nur bei genauester Einhaltung bestimmter Randbedingungen und Parameter gewährleistet:

Die beim vorbekannten Gebrauchsmuster eingesetzten Monopolstrahler sind sehr schmalbandig, was die Meßgenauigkeit reduziert, da zur Bestimmung der Meßgrößen Phasenverschiebung und Dämpfung nicht in einem größeren Frequenzbereich gemessen werden kann (sweepen), was die Genauigkeit vergrößert.

Die Durchführung der Messung beim vorbekannten Gebrauchsmuster mit den beiden Monopolantennen beruht im wesentlichen auf Rückstreuungen der Mikrowelle infolge der Kugelcharakteristik des Strahlungsfeldes dieser Monopolantennen. Dies führt zu einer sehr hohen Grunddämpfung (Dämpfung ohne Meßgut), die den dynamischen Meßbereich der Anordnung einschränkt.

Die Positionierung der Monopolantennen mit Hilfe des Tauchrohrs in dem Meßgut (Zuckersud/Zuckersaft) bedingt die Zuführung der Hochfrequenz über eine Koaxialleitung innerhalb des Tauchrohrs von der Außenwandung des Kochapparates bis zu den beiden Monopolantennen. Die Temperaturabhängigkeit dieser Koaxialkabel bringt einen erheblichen Nachteil insofern mit sich, als daß infolge der hohen Temperaturschwankungen im Kochapparat (ca. 75° beim Abkochen und ca. 125° beim Ausdampfen) die temperaturabhängige Kalibrierung der Vorrichtung streng definiert und überwacht werden muß. Hier ist also ein relativ hoher Aufwand erforderlich, um spätere Verfälschungen des Meßergebnisses zu vermeiden.

Gegenstand der Erfindung

Die wesentliche Aufgabe der Erfindung ist darin zu sehen, die vorbekannte Lösung so auszugestalten bzw. abzuändern, daß die die Meßgenauigkeit bestimmenden Parameter wie z.B. Grunddämpfung, Bandbreite wesentlich verbessert werden.

Eine weitere Aufgabe ist darin zu sehen, daß die Vorrichtung hinsichtlich Wartungsfreundlichkeit, Einfachheit des Aufbaus und Unempfindlichkeit gegen äußere Einflüsse verbessert wird.

Erfindungsgemäß wird diese Aufgabe gemäß dem kennzeichnenden Teil des Schutzanspruchs 1 gelöst.

Der Grundgedanke der Erfindung besteht im Einsatz einer unterbrochenen Hohlleiterführung mit sehr definierter Wellenführung und Wellenausbreitung; bei einer typischen kritischen Grenzfrequenz von 2,5 GHz ist eine solche Anordnung sehr breitbandig, so daß zur Bestimmung der erforderlichen Meßgrößen (Phasenverschiebung und Dämpfung) auch Sweep-Verfahren über einen größeren Frequenzbereich durchgeführt werden können, die die Genauigkeit des Meßergebnisses verbessern. Die Hohlleiterlösung erlaubt es auch, die Mikrowelle außerhalb des Meßgutes in den Hohlleiter einzukoppeln, so daß die Probleme mit temperaturbeaufschlagten Leitungszuführungen und die damit entstehenden Kalibrierungsprobleme vermieden werden.

Die Anordnung mit den Fenstern führt dazu, daß überwiegend die direkt (von Fenster zu Fenster) transmittierte Mikrowelle aufgenommen und ausgewertet wird, was zu einer minimierten Grunddämpfung mit einer Erhöhung des dynamischen Meßbereichs führt.

So haben die Meßergebnisse mit der erfindungsgemäßen Vorrichtung bereits gezeigt, daß in dem üblichen Temperaturbereich der Kochapparate bei der Bestimmung der Zuckerkonzentration keine signifikante Temperaturabhängigkeit dieser Hohlleiteranordnung besteht, Vergleichsmessungen mit in diesem Bereich seit langem eingeführten radiometrischen Meßmethoden zeigen einen hohen Grad von Übereinstimmung.

Die erfindungsgemäße Lösung erlaubt auch wesentliche vorteilhafte Ausgestaltungen, die Gegenstand von Unteransprüchen sind und mit denen beispielsweise die Anpassung auf das jeweilige Meßgut und die äußeren Parameter wie Form des Kochapparates und geometrische Randbedingungen optimiert werden kann.

Kurze Beschreibung der Zeichnungen

Ein bevorzugtes Ausführungsbeispiel der erfindungsgemäßen Vorrichtung wird nun anhand von Zeichnungen näher erläutert, es zeigen:

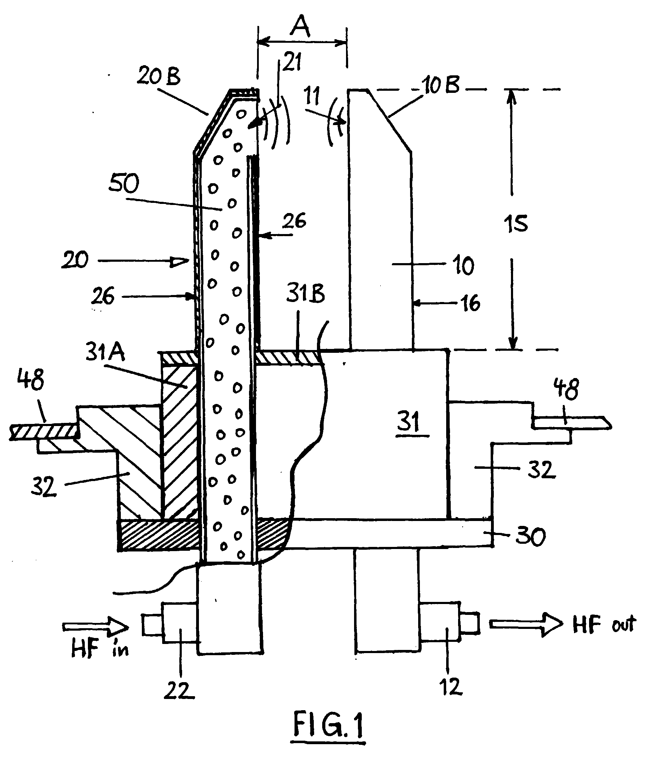

Figur 1:      Eine Ansichts-Teil-Schnittdarstellung durch ein bevorzugtes Ausführungsbeispiel des erfindungsgemäßen Mikrowellenapplikators,

Figur 2:      eine Ansicht eines Hohlleiters auf die Seitenfläche 10A in Figur 1, und

Figur 3:      ein Blockschaltbild eines Meßaufbaus unter Verwendung der erfindungsgemäßen Vorrichtung.

Beschreibung des bevorzugten Ausführungsbeispiels

Der in den Schutzansprüchen definierte Applikator besteht aus zwei parallel geführten Rund- oder Rechteckhohlleitern 10,20 aus elektrisch leitendem Material wie Edelstahl, die auf der Abschlußplatte 30 einer zylindrischen Edelstahldurchführung 31 eingeschweißt sind.

Die Abschlußplatte 30 ist auf einem Ringflansch 32 gehalten, der seinerseits auf einer Ausnehmung in der Wandung oder dem Deckel 48 des Kochapparates sitzt. Die Edelstahldurchführung 31 besteht aus einem Zylindermantel 31A mit oberem Abschlußdeckel 31B.

In den oberen Abschlußdeckel 31B sind rechteckige Aussparungen eingebracht, in denen die Hohlleiter 10, 20 dichtend gehalten sind. Die Edelstahldurchführung 31 dient somit zur sicheren Positionierung und Zentrierung der Hohlleiter auf der Abschlußplatte 30.

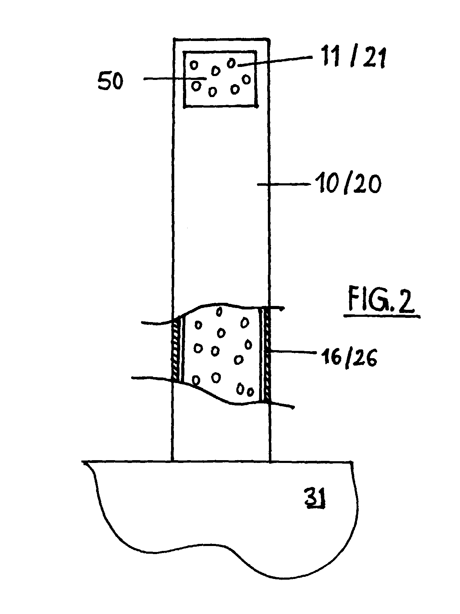

Auf der Meßgutseite weisen die Hohlleiter zwei gegenüberliegende Fensterausschnitte 11, 21 auf, aus denen die Mikrowelle aus- und eintritt (Fig. 2). An dem anderen Ende der Hohlleiter 10,20 außerhalb des Befestigungsflansches 30 befinden sich Durchführungsstekker 12, 22 zur Ein- und Auskopplung der Hochfrequenz. Wahlweise, je nach den geometrischen und physikalischen Meßbedingungen, können die Hohlleiter mit einem geeigneten Dieelektrikum 50 ausgegossen sein. Zusätzlich ist der in das Meßgut eintauchende Hohlleiterabschnitt 15 komplett mit einem für die Lebensmittelgüter verträglichen Schutzmantel 16,26 überzogen, dessen Wandstärke so gewählt wird, daß im Bereich der Aus- und Eintrittsfenster 11, 21 eine optimale Wellenanpassung an das Meßgut gewährleistet wird. Der Abstand A der Hohlleiter und der Fenster werden so gewählt, daß für das zu messende Medium eine möglichst große Sensitivität bezüglich der zu bestimmenden Meßgröße erreicht wird. Der Hohlleiterabschnitt 15 ist so angepaßt, daß zum einen die geometrischen Gegebenheiten, wie z.B. interne Heizspiralen im Behälter berücksichtigt, und zum anderen eine möglichst repräsentative Probe aus dem Meßgut erfaßt wird.

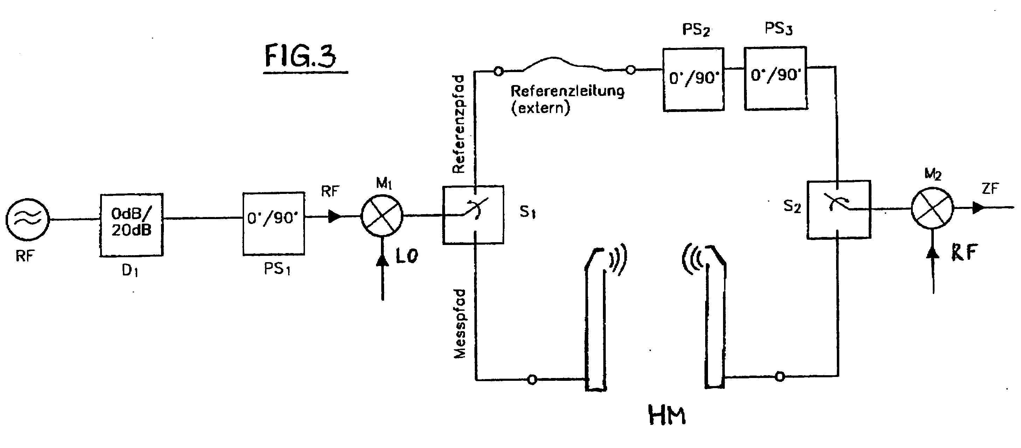

Zur Bestimmung des komplexen Transmissionsfaktors und damit der von den Meßguteigenschaften abhängigen Dämpfung und Phase ist der Meßplatz gemäß dem Prinzipblockschaltbild nach Fig. 3 aufgebaut. Die vom Synthesizer (RF) erzeugte Mikrowelle gelangt über ein umschaltbares Dämpfungsglied D1 und umschaltbaren Phasenschieber PS1 auf einen Mischer M1, in welchem eine Niederfrequenz (L0) von 50 kHz zugemischt wird. Danach folgt ein Schalter S1, welcher alternativ die Mikrowelle auf einen Referenzpfad und den Meßpfad mit der Hohlleiter-Meßeinrichtung HM weiterleitet. Beide Pfade werden wieder in einem zweiten Schalter S2 zusammengeführt und von dort die Mikrowelle in einem weiteren Mischer M2 wieder demoduliert. Die erzeugte Zwischenfrequenz ZF wird im 100 kHz-Takt mit Hilfe eines ADC's digitalisiert.

Der Referenzzweig dient der Kalibrierung und der Temperaturkompensation der HF-Kabel.

Die Messung der Probe erfolgt nun derart, daß zuerst eine Kalibriermessung z.B. mit Luft als Medium durchgeführt wird und Phase und Dämpfung als Bezugspunkte gespeichert werden. Danach wird mit der Meßprobe in einem vordefinierten Hochfrequenzbereich bei 22 Stützpunkten alternativ bei Null Grad und 90 Grad Phasenverschiebung die Zwischenfrequenz digitalisiert. Hiermit werden Realteil und Imaginärteil des Transmissionsfaktors ermittelt, wobei der Betrag der komplexen Größe der Dämpfung und der Winkel zwischen komplexer Größe und Realteilachse der Phasenverschiebung entspricht. Die Mittelung über die Frequenzstützpunkte erfolgt derart, daß bei der Dämpfung das arithmetische Mittel gebildet und bei der Phase nach einer linearen Regression der Bestfitwert an der Stelle der Mittenfrequenz als Phasenverschiebung gewählt wird. Phasenverschiebung und Dämpfung sind dann das zu ermittelnde Maß für die Zuckerkonzentration des Meßgutes.

**Patentansprüche**

1. Vorrichtung zur Transmissionsmessung mit Hilfe von Mikrowellen zur kontinuierlichen Bestimmung der Konzentration von Zuckersaft während des Kochprozesses in einem Kochapparat beim Kristallisationsvorgang, mit zwei in das Meßgut eingebrachten, als Sende- und Empfangsantenne arbeitenden Einrichtungen, dadurch gekennzeichnet, daß zwei in das Meßgut eintauchende Hohlleiter (10,20) vorgesehen sind, die zur Abstrahlung der Mikrowellen geeignete Fenster (11,21) aufweisen.

2. Vorrichtung nach Anspruch 1, dadurch gekennzeichnet, daß die Fenster (11,21) in zueinander zeigende Flächen (10A,20A) oder Flächenbereiche der Hohlleiter (10,20) eingebracht sind.

3. Vorrichtung nach Anspruch 2, dadurch gekennzeichnet, daß die Fenster (11,21) auf gleicher Höhe angeordnet sind.

4. Vorrichtung nach Anspruch 1-3, dadurch gekennzeichnet, daß die Fenster (11,21) im Endbereich der Hohlleiter (10,20) angebracht sind.

5. Vorrichtung nach Anspruch 4, dadurch gekennzeichnet, daß der Endbereich der Hohlleiter (10,20) zum jeweiligen Fenster (11,21) zeigende, dachförmige Abschrägungen (10B,20B) aufweist.

6. Vorrichtung nach Anspruch 1, dadurch gekennzeichnet, daß die Hohlleiter (10,20) aus der Außenwandung des Kochapparates hinausragen, wo die Einkopplung der Mikrowelle über Stecker (12,22) erfolgt.

7. Vorrichtung nach Anspruch 1, dadurch gekennzeichnet, daß die Hohlleiter (10,20) rechteckigen oder kreisförmigen Querschnitt haben.

8. Vorrichtung nach Anspruch 1, dadurch gekennzeichnet, daß die Hohlleiter (10,20) aus elektrisch leitendem Material wie z. B. Edelstahl sind.

9. Vorrichtung nach Anspruch 1, dadurch gekennzeichnet, daß die Hohlleiter (10,20) ganz oder teilweise mit einem Dielektrikum (50) ausgegossen sind.

10. Vorrichtung nach Anspruch 1, dadurch gekennzeichnet, daß die Außenseite der in das Meßgut eintauchenden Hohlleiter mit einem Schutzmantel (16,26) überzogen ist, dessen Wandstärke auf die Optimierung der Wellenanpassung an das Meßgut im Bereich der Fenster (11,21) ausgelegt ist.

11. Vorrichtung nach Anspruch 1, dadurch gekennzeichnet, daß der Abstand (A) der Fenster der Hohlleiter (10,20), die Fläche der Fenster (11,21) und der in das Meßgut eintauchende Hohlleiterabschnitt (15) auf die Optimierung der Meßempfindlichkeit ausgelegt sind.

12. Vorrichtung nach Anspruch 1, dadurch gekennzeichnet, daß der Querschnitt der Hohlleiter so gewählt ist, daß die Transmission von Mikrowellen im Frequenzbereich von 1 bis 12 GHz im wesentlichen dämpfungsfrei erfolgt.

FIG.1

50

11 / 21

10 / 20

FIG. 2

16/26

31

FIG.3

Europäisches
Patentamt

**EUROPÄISCHER RECHERCHENBERICHT**

Nummer der Anmeldung

EP 98 12 1810

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | US 4 641 083 A (OHNO JIRO ET AL) 3. Februar 1987 * Anspruch 3 * --- | 1-4 | G01N22/00 |
| D,Y | DE 296 17 488 U (PRO M TEC THEISEN GMBH) 15. Mai 1997 * das ganze Dokument * --- | 1-4 | |
| A | GB 2 247 081 A (MASTER CHEMICAL CORP) 19. Februar 1992 * Anspruch 1 * ----- | 1 | |

RECHERCHIERTE SACHGEBIETE (Int.Cl.6)

G01N

Der vorliegende Recherchenbericht wurde für alle Patentansprüche erstellt

| Recherchenort | Abschlußdatum der Recherche | Prüfer |
|---|---|---|
| DEN HAAG | 10. März 1999 | HULNE S.L. |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument
 
& : Mitglied der gleichen Patentfamilie, übereinstimmendes
  Dokument

EPO FORM 1503 03 82 (P04C03)

**ANHANG ZUM EUROPÄISCHEN RECHERCHENBERICHT**
**ÜBER DIE EUROPÄISCHE PATENTANMELDUNG NR.**   EP 98 12 1810

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-03-1999

| Im Recherchenbericht angeführtes Patentdokument | Datum der Veröffentlichung | Mitglied(er) der Patentfamilie | Datum der Veröffentlichung |
|---|---|---|---|
| US 4641083 A | 03-02-1987 | JP 1453517 C | 10-08-1988 |
| | | JP 60013009 A | 23-01-1985 |
| | | JP 62059163 B | 09-12-1987 |
| | | JP 1038842 B | 16-08-1989 |
| | | JP 1556318 C | 23-04-1990 |
| | | JP 59026038 A | 10-02-1984 |
| | | JP 1044042 B | 25-09-1989 |
| | | JP 1564946 C | 25-06-1990 |
| | | JP 59027604 A | 14-02-1984 |
| | | JP 1339638 C | 29-09-1986 |
| | | JP 59038313 A | 02-03-1984 |
| | | JP 61006126 B | 24-02-1986 |
| | | JP 1369468 C | 25-03-1987 |
| | | JP 59083708 A | 15-05-1984 |
| | | JP 61036563 B | 19-08-1986 |
| | | CA 1200903 A | 18-02-1986 |
| | | EP 0101219 A | 22-02-1984 |
| DE 29617488 U | 15-05-1997 | KEINE | |
| GB 2247081 A | 19-02-1992 | US 4767982 A | 30-08-1988 |
| | | GB 2247080 A,B | 19-02-1992 |
| | | DE 3741577 A | 15-12-1988 |
| | | GB 2205407 A,B | 07-12-1988 |
| | | JP 63311154 A | 19-12-1988 |

EPO FORM P0461